# EUROPEAN PATENT APPLICATION

(11) **EP 3 725 321 A1**
(43) Date of publication of application: **21.10.2020**
(21) Application number: 18888922.4
(22) Date of filing: 12.12.2018
(51) Int. Cl.: A61K 35/745, A23L 33/135, A61P 1/02, A61P 1/04, A61P 15/06, A61P 29/00, A61P 31/04, A61P 35/00, C12N 1/20

(54) **COMPOSITION CONTAINING BACTERIUM BELONGING TO GENUS BIFIDOBACTERIUM AS ACTIVE INGREDIENT**

(30) Priority: 12.12.2017 JP 2017237465
(71) Applicant: Morinaga Milk Industry Co., Ltd., Minato-ku Tokyo 108-8384 (JP)
(72) Inventor: YOSHIMOTO, Shin, Zama-shi, Kanagawa 252-8583 (JP); ODAMAKI, Toshitaka, Zama-shi, Kanagawa 252-8583 (JP)
(74) Representative: Vossius & Partner Patentanwälte Rechtsanwälte mbB
(86) International application number: PCT/JP2018/045755
(87) International publication number: WO 2019/117212

(57) **Abstract**

The present invention addresses the problem of providing: a composition for preventing the adsorption of *Fusobacterium* to mucosal cells; and a pharmaceutical composition and a food or beverage composition, for preventing or treating a disease or symptom caused by inflammation of mucosal cells and selected from the group consisting of ulcerative colitis, colon cancer, esophageal cancer, premature delivery, and periodontosis. The problem is solved by: a composition for preventing the adsorption of *Fusobacterium* to mucosal cells, containing *Bifidobacterium* as an active ingredient; and a pharmaceutical composition and a food or beverage composition each containing *Bifidobacterium* as an active ingredient, for preventing or treating a disease or symptom caused by inflammation of mucosal cells and selected from the group consisting of ulcerative colitis, colon cancer, esophageal cancer, premature delivery, and periodontosis.

## Description

### Technical Field

The present invention relates to a composition for preventing the adsorption of *Fusobacterium* to mucosal cells. The invention also relates to a pharmaceutical composition and a food or beverage composition each containing *Bifidobacterium* as an active ingredient, for preventing or treating a disease or symptom caused by inflammation of mucosal cells and selected from the group consisting of ulcerative colitis, colon cancer, esophageal cancer, premature delivery, and periodontosis.

### Background Art

Some bacteria belonging to the genus *Bifidobacterium* are known to be effective in the prevention, treatment, alleviation, or the like of diseases or symptoms in humans.

For example, *Bifidobacterium bifidum* G9-1 is known to be effective in preventing or treating 5-fluorouracil-induced intestinal diseases (Patent Literature 1).

In addition, bacteria belonging to *Bifidobacterium breve,* bacteria belonging to *Bifidobacterium longum,* and the like are known to be effective in preventing or alleviating arthritis (Patent Literature 2).

However, it is not known that *Bifidobacterium* are effective in preventing or treating diseases or symptoms caused by inflammation of mucosal cells, such as ulcerative colitis, colon cancer, esophageal cancer, premature delivery, and periodontosis.

Against this background, there has been a need for components effective in preventing or treating diseases or symptoms caused by inflammation of mucosal cells, such as ulcerative colitis, colon cancer, esophageal cancer, premature delivery, and periodontosis.

Meanwhile, *Fusobacterium* are normal inhabitants in the human oropharynx and known to be associated with the onset of periodontosis (Patent Literature 3). Further, in recent years, it has been pointed out that *Fusobacterium* are associated with ulcerative colitis, colon cancer, esophageal cancer, and premature delivery.

It has been reported that *Fusobacterium* are abundant in the colon of a patient with ulcerative colitis or colon cancer, and that they are capable of adsorbing to or invading into the colonic mucosal epithelium (Non Patent Literature 1).

In addition, it has been reported that human colon cancer cells express the sugar chain Gal-GalNAc, and that *Fusobacterium* adsorb to the Gal-GalNAc through Fap2, which is their own Gal-GalNAc-binding lectin (Non Patent Literature 2).

In addition, it has been reported that as a result of checking the presence of *Fusobacterium* in 325 cases of esophageal cancer tissues, the presence of *Fusobacterium* was confirmed in 74 cases, where the prognosis of esophageal cancer was poor (Non Patent Literature 3).

In addition, it has been reported that Fap2 that *Fusobacterium* have is a galactose-inhibiting adhesion factor associated with premature delivery (Non Patent Literature 4).

Against this background, there has been a need for the development of a composition for preventing the adsorption of *Fusobacterium* to mucosal cells.

### Citation List

### Patent Literature

Patent Literature 1: JP-A-2017-088540
Patent Literature 2: JP-A-2012-158568
Patent Literature 3: JP-A-2016-192950

### Non Patent Literature

Non Patent Literature 1: Journal of Intestinal Microbiology, 27, 169-179 (2013)
Non Patent Literature 2: Cell Host Microbe, 20, 215-225 (2016)
Non Patent Literature 3: Clin. Cancer Res., 22 (22) (2016)
Non Patent Literature 4: Infect. Immun., 83 (3), 1104-13, (2015)

### Summary of Invention

### Technical Problems

An object of the invention is to provide a composition for preventing the adsorption of *Fusobacterium* to mucosal cells. Another object of the invention is to provide a pharmaceutical composition and a food or beverage composition, for preventing or treating a disease or symptom caused by inflammation of mucosal cells and selected from the group consisting of ulcerative colitis, colon cancer, esophageal cancer, premature delivery, and periodontosis.

### Solution to Problems

The present inventors have found that *Bifidobacterium* prevent the adsorption of *Fusobacterium* to mucosal cells, and also that *Bifidobacterium* are effective in preventing or treating a disease or symptom caused by inflammation of mucosal cells and selected from the group consisting of ulcerative colitis, colon cancer, esophageal cancer, premature delivery, and periodontosis, and thus accomplished the invention.

The present invention is a composition for preventing the adsorption of *Fusobacterium* to mucosal cells, containing *Bifidobacterium* as an active ingredient.

According to a preferred embodiment of the composition for preventing adsorption, the mucosal cells are colon epithelial cells, mucosal epithelial cells of the esophagus, vascular endothelial cells of the uterus, or mucosal cells of the gingiva.

According to a preferred embodiment of the composition for preventing adsorption, the *Bifidobacterium* are at least one kind of bacteria selected from the group consisting of bacteria belonging to *Bifidobacterium longum subspecies longum,* bacteria belonging to *Bifidobacterium bifidum,* and bacteria belonging to *Bifidobacterium longum subspecies infantis.*

According to a preferred embodiment of the composition for preventing adsorption, the bacteria belonging to *Bifidobacterium longum subspecies longum* are at least one kind of bacteria selected from the group consisting of *Bifidobacterium longum subspecies longum* NITE BP-02572, NITE BP-02573, and NITE BP-02574.

According to a preferred embodiment of the composition for preventing adsorption, the bacteria belonging to *Bifidobacterium bifidum* are at least one kind of bacteria selected from the group consisting of *Bifidobacterium bifidum* NITE BP-1252, NITE BP-02570, and NITE BP-02571.

According to a preferred embodiment of the composition for preventing adsorption, the bacteria belonging to *Bifidobacterium longum subspecies infantis* are *Bifidobacterium longum subspecies infantis* ATCC 15697.

In addition, the present invention provides apharmaceutical compositions containing *Bifidobacterium* as an active ingredient, for preventing or treating a disease or symptom caused by inflammation of mucosal cells and selected from the group consisting of ulcerative colitis, colon cancer, esophageal cancer, premature delivery, and periodontosis.

According to a preferred embodiment of the pharmaceutical composition, the inflammation of mucosal cells is inflammation of mucosal cells involving *Fusobacterium.*

According to a preferred embodiment of the pharmaceutical composition, the *Bifidobacterium* are at least one kind of bacteria selected from the group consisting of bacteria belonging to *Bifidobacterium longum subspecies longum,* bacteria belonging to *Bifidobacterium bifidum,* and bacteria belonging to *Bifidobacterium longum subspecies infantis.*

According to a preferred embodiment of the pharmaceutical composition, the bacteria belonging to *Bifidobacterium longum subspecies longum* are at least one kind of bacteria selected from the group consisting of *Bifidobacterium longum subspecies longum* NITE BP-02572, NITE BP-02573, and NITE BP-02574.

According to a preferred embodiment of the pharmaceutical composition, the bacteria belonging to *Bifidobacterium bifidum* are at least one kind of bacteria selected from the group consisting of *Bifidobacterium bifidum* NITE BP-1252, NITE BP-02570, and NITE BP-02571.

According to a preferred embodiment of the pharmaceutical compositions, the bacteria belonging to *Bifidobacterium longum subspecies infantis* are *Bifidobacterium longum subspecies infantis* ATCC 15697.

In addition, the present invention provides a food or beverage composition containing *Bifidobacterium* as an active ingredient, for preventing or treating a disease or symptom caused by inflammation of mucosal cells and selected from the group consisting of ulcerative colitis, colon cancer, esophageal cancer, premature delivery, and periodontosis.

According to a preferred embodiment of the food or beverage composition, the inflammation of mucosal cells is inflammation of mucosal cells caused by *Fusobacterium.*

According to a preferred embodiment of the food or beverage composition, the *Bifidobacterium* are at least one kind of bacteria selected from the group consisting of bacteria belonging to *Bifidobacterium longum subspecies longum,* bacteria belonging to *Bifidobacterium bifidum,* and bacteria belonging to *Bifidobacterium longum subspecies infantis.*

According to a preferred embodiment of the food or beverage composition, the bacteria belonging to *Bifidobacterium longum subspecies longum* are at least one kind of bacteria selected from the group consisting of *Bifidobacterium longum subspecies longum* NITE BP-02572, NITE BP-02573, and NITE BP-02574.

According to a preferred embodiment of the food or beverage composition, the bacteria belonging to *Bifidobacterium bifidum* are at least one kind of bacteria selected from the group consisting of *Bifidobacterium bifidum* NITE BP-1252, NITE BP-02570, and NITE BP-02571.

According to a preferred embodiment of the food or beverage composition, the bacteria belonging to *Bifidobacterium longum subspecies infantis* are *Bifidobacterium longum subspecies infantis* ATCC 15697.

In addition, the present invention provides *Bifidobacterium longum subspecies longum* NITE BP-02572, NITE BP-02573, and NITE BP-02574 and *Bifidobacterium bifidum* NITE BP-02570 and NITE BP-02571.

### Effects of Invention

The present invention enables the provision of a composition for preventing the adsorption of *Fusobacterium* to mucosal cells. In addition, the present invention enables the provision of a pharmaceutical composition and a food or beverage composition, for preventing or treating a disease or symptom caused by inflammation of mucosal cells and selected from the group consisting of ulcerative colitis, colon cancer, esophageal cancer, premature delivery, and periodontosis.

### Description of Embodiments

Hereinafter, the present invention will be described in detail.

The composition of the present invention contains *Bifidobacterium* as an active ingredient.

In addition, the composition of the invention encompasses the following embodiment 1 and 2.

Embodiment 1: A composition for preventing the adsorption of *Fusobacterium* to mucosal cells, containing *Bifidobacterium* as an active ingredient.

Embodiment 2: A composition containing *Bifidobacterium* as an active ingredient, for preventing or treating a disease or symptom caused by inflammation of mucosal cells and selected from the group consisting of ulcerative colitis, colon cancer, esophageal cancer, premature delivery, and periodontosis.

As compositions according to the above embodiment 2, for example, a pharmaceutical composition, a food or beverage composition, and the like can be mentioned.

It has been reported that *Bifidobacterium* exert various physiological functions, and that such functions are due to the growth of the bacteria in the intestine or substances that the bacteria produce in the intestine (e.g., acetic acid, etc.). Therefore, according to the present invention, the generally supposed, highly safe efficacy of *Bifidobacterium* can be expected across a wide range of age groups, and such efficacy can be realized for a wide range of intended uses (for foods and beverages, for pharmaceuticals, for animal feeds, etc.).

In addition, the *Bifidobacterium* of the present invention can be expected to have probiotic effects, and thus can also be used for the purposes of health promotion, diet improvement, intestinal environment improvement, intestinal infection prevention or treatment, immune activity recovery or enhancement, and the like.

*Bifidobacterium* are Gram-positive, obligate anaerobic bacilli.

The *Bifidobacterium* are not particularly limited, and examples thereof include bacteria belonging to *Bifidobacterium longum subspecies longum,* bacteria belonging to *Bifidobacterium bifidum,* and bacteria belonging to *Bifidobacterium longum subspecies infantis.*

Incidentally, *Bifidobacterium longum subspecies longum* is sometimes simply referred to as *"Bifidobacterium longum".* In addition, *Bifidobacterium longum subspecies infantis* is sometimes simply referred to as *"Bifidobacterium infantis".*

Specific examples thereof include *Bifidobacterium longum subspecies longum* NITE BP-02572, *Bifidobacterium longum subspecies longum* NITE BP-02573, *Bifidobacterium longum subspecies longum* NITE BP-02574, *Bifidobacterium longum subspecies longum* ATCC BAA-999, *Bifidobacterium bifidum* NITE BP-1252, *Bifidobacterium bifidum* NITE BP-02570, *Bifidobacterium bifidum* NITE BP-02571, and *Bifidobacterium longum subspecies infantis* ATCC 15697, as well as bacterial cells of mutants thereof.

The above *Bifidobacterium longum subspecies longum* NITE BP-02572, NITE BP-02573, and NITE BP-02574 have all been internationally deposited to National Institute of Technology and Evaluation (NITE), Patent Microorganisms Depositary (Room 122, 2-5-8 Kazusakamatari, Kisarazu, Chiba 292-0818), on November 13, 2017, under the Budapest Treaty (the accession numbers are NITE BP-02572, NITE BP-02573, and NITE BP-02574, respectively).

In addition, *Bifidobacterium longum subspecies longum* ATCC BAA-999 is available from American Type Culture Collection (address: 12301 Parklawn Drive, Rockville, Maryland 20852, United States of America).

In addition, *Bifidobacterium bifidum* NITE BP-1252 has been internationally deposited to National Institute of Technology and Evaluation (NITE), Patent Microorganisms Depositary (Room 122, 2-5-8 Kazusakamatari, Kisarazu, Chiba 292-0818), on February 23, 2012, under the Budapest Treaty (the accession number is NITE BP-1252).

In addition, *Bifidobacterium bifidum* NITE BP-02570 and NITE BP-02571 have both been internationally deposited to National Institute of Technology and Evaluation (NITE), Patent Microorganisms Depositary (Room 122, 2-5-8 Kazusakamatari, Kisarazu, Chiba 292-0818), on November 13, 2017, under the Budapest Treaty (the accession numbers are NITE BP-02570 and NITE BP-02571, respectively).

In addition, *Bifidobacterium longum subspecies infantis* ATCC 15697 is available from American Type Culture Collection (address: 12301 Parklawn Drive, Rockville, Maryland 20852, United States of America).

*Bifidobacterium longum subspecies longum* NITE BP-02572 is not limited to the above bacteria, and may also be bacteria substantially equivalent to the above bacteria. "Bacteria substantially equivalent to the above bacteria" are bacteria that are classified as *Bifidobacterium longum subspecies longum* and capable of realizing the intended uses of the above embodiments 1 and 2. Further, they have a 16SrRNA gene base sequence having a homology of preferably 98% or more, still more preferably 99% or more, and more preferably 100% with the 16SrRNA gene base sequence of the above bacteria, and preferably also have the same mycological properties as the above bacteria. In addition, bacteria classified as *Bifidobacterium longum subspecies longum* also include mutant strains and genetically modified strains of such bacteria as parent strains.

This applies also to the other bacterial cells described above.

In addition, in the present invention, it is necessary to use at least one kind of *Bifidobacterium,* and it is also possible to use two or more kinds.

The 16SrRNA gene base sequences of *Bifidobacterium longum subspecies longum* NITE BP-02572, NITE BP-02573, and NITE BP-02574 are represented as SEQ ID NOs: 1, 2, and 3, respectively. Each bacterial cells thereof have, as a result of comparison with *Bifidobacterium longum subspecies longum* JCM 1217 (the most closely related species on the DNA database (BLAST)) using a sequence length of 1,535 bp, homologies of 99.8%, 99.6%, and 99.8%, respectively. Therefore, it has been judged that all the bacterial cells are bacteria classified as *Bifidobacterium longum subspecies longum.*

The 16SrRNA gene base sequences of *Bifidobacterium bifidum* NITE BP-02570 and NITE BP-02571 are represented by SEQ ID NOs: 4 and 5, respectively. Each bacterial cells thereof have, as a result of comparison with *Bifidobacterium bifidum* JCM 1255 (the most closely related species on the DNA database (BLAST)) using a sequence length of 1,422 bp, a homology of 99.9%. Therefore, it has been judged that all the bacterial cells are bacteria classified as *Bifidobacterium bifidum.*

*Bifidobacterium longum subspecies longum* JCM 1217 and *Bifidobacterium bifidum* JCM 1255 are available from Japan Collection of Microorganisms (RIKEN BioResource Center, Microbe Division, zip: 305-0074, address: 3-1-1 Koyadai, Tsukuba, Ibaraki).

The *Bifidobacterium* used in the present invention have, in the above embodiment 1, a preventing action on the adsorption of *Fusobacterium* to mucosal cells. In addition, in the above embodiment 2, the *Bifidobacterium* preferably have a preventing action on the adsorption of *Fusobacterium* to mucosal cells.

The preventing action on the adsorption of *Fusobacterium* to mucosal cells means an action of reducing the number of *Fusobacterium* adsorbed to mucosal cells in a subject, and means that when *Bifidobacterium* are ingested by (including the case of "administered to") a subject, the number of *Fusobacterium* adsorbed to mucosal cells is smaller as compared with when the bacteria are not ingested (including the case of "not administered").

The *Bifidobacterium* can be easily grown by culturing such bacteria. The culturing method is not particularly limited as long as the bacteria can grow, and a method commonly used for culturing *Bifidobacterium* (bifidobacteria) can be suitably modified if necessary and used. For example, the culturing temperature may be 25 to 50°C, and is preferably 30 to 40°C. In addition, culture is preferably performed under anaerobic conditions. For example, the bacteria may be cultured while passing an anaerobic gas, such as carbon dioxide. In addition, culture under microaerophilic conditions, such as liquid stationary culture, is also possible.

The medium used for culture is not particularly limited, and a medium commonly used for culturing *Bifidobacterium* can be suitably modified if necessary and used. That is, as carbon sources, for example, saccharides such as galactose, glucose, fructose, mannose, cellobiose, maltose, lactose, sucrose, trehalose, starch, starch hydrolysates, and blackstrap molasses can be used according to the utilization. As nitrogen sources, for example, ammonium salts and nitrate salts, such as ammonia, ammonium sulfate, ammonium chloride, and ammonium nitrate, can be used. In addition, as inorganic salts, for example, sodium chloride, potassium chloride, potassium phosphate, magnesium sulfate, calcium chloride, calcium nitrate, manganese chloride, ferrous sulfate, and the like can be used. In addition, organic components such as peptone, soybean flour, defatted soybean cake, meat extracts, and yeast extracts may also be used. In addition, as a prepared medium, an MRS medium can be preferably used, for example.

As the *Bifidobacterium,* the culture product obtained after culture may be directly used, or may also be diluted or concentrated and used. It is also possible to use bacterial cells recovered from the culture product. In addition, unless the effects of the present invention are impaired, culture may be followed by various additional operations such as heating and lyophilizing. Incidentally, the *Bifidobacterium* may be live or killed. As killed bacteria, killed bacteria sterilized by heating or the like can be mentioned.

Next, the *Fusobacterium* in the present invention are Gram-negative anaerobic bacilli, and are bacteria that produce high-concentration butyric acid as a metabolic product of intestinal fermentation. In the above embodiment 1, the *Fusobacterium* in the present invention are not particularly limited as long as they adsorb to mucosal cells, while in the above embodiment 2, the bacteria are preferably involved in inflammation of mucosal cells. To be involved in inflammation of mucosal cells means, for example, adsorbing to mucosal cells to cause inflammation of the mucosal cells.

Examples thereof include bacteria belonging to *Fusobacterium nucleatum,* bacteria belonging to *Fusobacterium gonidiaformans,* bacteria belonging to *Fusobacterium naviforme,* bacteria belonging to *Fusobacterium periodonticum,* bacteria belonging to *Fusobacterium necrophorum,* bacteria belonging to *Fusobacterium varium,* bacteria belonging to *Fusobacterium canifelinum,* bacteria belonging to *Fusobacterium equinum,* bacteria belonging to *Fusobacterium gastrosuis,* bacteria belonging to *Fusobacterium hwasookii,* bacteria belonging to *Fusobacterium massiliense,* bacteria belonging to *Fusobacterium mortiferum,* bacteria belonging to *Fusobacterium necrogenes,* bacteria belonging to *Fusobacterium perfoetens,* bacteria belonging to *Fusobacterium russii,* bacteria belonging to *Fusobacterium simiae,* and bacteria belonging to *Fusobacterium ulcerans.*

Specific examples thereof include *Fusobacterium nucleatum subspecies nucleatum* ATCC 23726, *Fusobacterium gonidiaformans* ATCC 25563, *Fusobacterium naviforme* ATCC 25832, *Fusobacterium periodonticum* ATCC 33693, *Fusobacterium necrophorum* ATCC 25286, *Fusobacterium varium* ATCC 8501, *Fusobacterium canifelinum* ATCC BAA-689, *Fusobacterium mortiferum* ATCC 25557, *Fusobacterium necrogenes* ATCC 25556, *Fusobacterium perfoetens* ATCC 29250, *Fusobacterium russii* ATCC 25533, *Fusobacterium simiae* ATCC 33568, and *Fusobacterium ulcerans* ATCC 49185, as well as bacterial cells of mutant strains thereof.

The bacterial cells are all available from American Type Culture Collection (address: 12301 Parklawn Drive, Rockville, Maryland 20852, United States of America).

*Fusobacterium nucleatum subspecies nucleatum* ATCC 23726 is not limited to the above bacteria, and may also be bacteria substantially equivalent to the above bacteria. "Bacteria substantially equivalent to the above bacteria" are bacteria classified as *Fusobacterium nucleatum subspecies nucleatum,* and are bacteria capable of adsorbing to mucosal cells in the above embodiment 1, while are preferably bacteria involved in inflammation of mucosal cells in the above embodiment 2. Further, they have a 16SrRNA gene base sequence having a homology of preferably 98% or more, still more preferably 99% or more, and more preferably 100% with the 16SrRNA gene base sequence of the above bacteria, and preferably also have the same mycological properties as the above bacteria. To be involved in inflammation of mucosal cells means, for example, adsorbing to mucosal cells to cause inflammation of the mucosal cells. In addition, bacteria classified as *Fusobacterium nucleatum subspecies nucleatum* also include mutant strains and genetically modified strains of such bacteria as parent strains.

This applies also to the other bacterial cells described above.

In addition, in the present invention, it is necessary to use at least one kind of *Fusobacterium,* and it is also possible to use two or more kinds.

The mucosal cells in the present invention are, in the case where the mucosal cells are untreated, that is, not treated with *Bifidobacterium,* for example, in the case where they are not allowed to react (coexist) with *Bifidobacterium,* in the above embodiment 1, not particularly limit as long as *Fusobacterium* adsorb thereto *Bifidobacterium,* while in the above embodiment 2, the mucosal cells are not particularly limited, but are preferably cells that develop inflammation as a result of the adsorption of *Fusobacterium* in such a case. In any embodiment, for example, colon epithelial cells, vascular endothelial cells of the uterus, mucosal epithelial cells of the esophagus, and mucosal cells of the gingiva can be mentioned.

The preventing effect of the *Bifidobacterium* of the present invention on the adsorption of *Fusobacterium* to mucosal cells can be confirmed by the following method described in the Examples, for example.

A colon cancer cell strain HCT116 is allowed to react (coexist) with *Bifidobacterium,* and then the HCT116 is allowed to react (coexist) with FITC (fluorescein isothiocyanate)-labeled *Fusobacterium nucleatum* ATCC 23726. FITC-positive cells are regarded as cells having adsorbed thereto FITC-labeled ATCC 23726, and the FITC positive rate in the case where HCT116 unreacted (not coexisted) with *Bifidobacterium* is allowed to react (coexist) with ATCC 23726 is taken as a control (100%). Then, the effect is confirmed by a decrease in the proportion of positive cells (FITC positive rate).

The composition of the present invention is a concept that encompasses a mixture, regardless of whether the components of the composition are uniform or nonuniform.

The composition for preventing the adsorption of *Fusobacterium* to mucosal cells according to the above embodiment 1 of the present invention can be utilized as a food or beverage composition for preventing adsorption or a pharmaceutical composition for preventing adsorption.

In addition, the composition according to the above embodiment 1 can be utilized as a food or beverage composition or pharmaceutical composition for preventing or treating a disease or symptom caused by *Fusobacterium.* In addition, the food or beverage composition according to the above embodiment 1 has a preventing action on the adsorption of *Fusobacterium* to mucosal cells, and thus can be preferably used for preventing or treating a disease or symptom that can be prevented or treated by preventing the adsorption of *Fusobacterium* to mucosal cells. "Treatment" also includes alleviation and improvement. Specific examples of such applications include the prevention and treatment of diseases and symptoms such as ulcerative colitis, colon cancer, esophageal cancer, premature delivery, and periodontosis. In addition, "premature delivery" herein is defined as a "symptom".

The composition for preventing or treating a disease or symptom caused by inflammation of mucosal cells and selected from the group consisting of ulcerative colitis, colon cancer, esophageal cancer, premature delivery, and periodontosis according to the above embodiment 2 can be utilized as a food or beverage composition as described above. The inflammation of mucosal cells is preferably inflammation of mucosal cells caused by *Fusobacterium,* and is more preferably inflammation of mucosal cells caused by the adsorption of *Fusobacterium* to mucosal cells.

The food or beverage composition of the present invention contains *Bifidobacterium.* The food or beverage composition may be in the form of a liquid, paste, gel-like solid, powder, or the like, and may also be a food or beverage, such as a tablet confectionery or a liquid food. In addition, examples thereof also include flour products such as bread, macaroni, spaghetti, noodles, cake mixes, seasoned flour for fried chicken, and bread crumbs; instant foods such as instant noodles, instant noodles in cups, retort-packaged/cooked foods, canned cooked foods, microwave foods, instant soups/stews, instant miso soups/clear soups, canned soups, freeze-dried foods, and other instant foods; processed agricultural products such as canned agricultural products, canned fruits, jams/marmalades, pickles, simmered legumes, dried agricultural products, and cereals (processed grain products) ; processed fishery products such as canned fishery products, fish meat hams/sausages, pasted fishery products, fishery delicacies, and *tsukudani* (preservable food boiled down in sweetened soy sauce); processed livestock products such as canned/pasted livestock products and livestock meat hams/sausages; milk/dairy products such as processed milks, milk beverages, yogurts, lactic acid bacteria beverages, cheeses, ice creams, modified milk powders, creams, and other dairy products; oils and fats such as butter, margarine, and vegetable oils; basic seasonings such as soy sauce, miso, sauces, processed tomato seasonings, *mirin* (sweet cooking sake), and vinegars; composite seasonings/foods such as cooking mixes, curry bases, sauces, dressings, noodle seasonings, spices, and other composite seasonings; frozen foods such as frozen raw foods, frozen semi-cooked foods, and frozen cooked foods; confectioneries such as caramels, candies, gummy candies, chewing gums, chocolates, cookies, biscuits, cakes, pies, snacks, crackers, Japanese sweets, rice confectioneries, bean confectioneries, dessert confectioneries, jellies, and other confectioneries; fancy drinks such as carbonated beverages, natural fruit juices, fruit juice beverages, fruit juice-containing soft beverages, fruit pulp beverages, granule-containing fruit beverages, vegetable-based beverages, soy milk, soy milk beverages, coffee beverages, tea beverages, powdered beverages, concentrated beverages, sports beverages, nutritional beverages, alcoholic beverages, and other fancy drinks; other commercially available foods such as baby foods, *furikake* (seasoning powder for rice), and *ochazuke-nori* (seasoning powder for rice soup); nursery formulas; enteral foods; special purpose foods and foods with health claims (foods for specified health uses, foods with nutrient function claims, foods with function claims); and nutritional supplementary foods.

In addition, the food or beverage composition may also be a supplement, such as a tablet-shaped supplement. In the case where the composition is a supplement, the *Bifidobacterium* can be ingested without being affected by other foods in terms of the daily food intake and calorie intake.

The food or beverage composition of the present invention can be produced by adding *Bifidobacterium* to raw materials for ordinary foods and beverages, and can be produced in the same manner as for ordinary foods and beverages, except for adding *Bifidobacterium.* The addition of *Bifidobacterium* may be made in any step of the production of the food or beverage composition. In addition, the food or beverage composition may also be produced through a step of fermentation by the added *Bifidobacterium.* Examples of such food or beverage compositions include bifidobacteria beverages and fermented milks.

As raw materials for the food or beverage composition, raw materials used for ordinary foods and beverages can be used. The produced food or beverage compositions can be orally ingested.

The food or beverage composition of the present invention also contains raw materials for producing the food or beverage composition, food additives, and the like added to the food or beverage composition in the course of or after the production of the food or beverage composition. For example, the *Bifidobacterium* in the invention can be used as a starter for fermented milk production. In addition, the *Bifidobacterium* in the present invention can also be added later to the produced fermented milk.

In addition, unless the effects of the present invention are impaired, in the food or beverage composition of the present invention, a component having probiotic effects or a component that aids probiotic effects, now known or later discovered, can be used. For example, the food or beverage composition of the present invention can be prepared by blending, with the *Bifidobacterium* of the invention, the following components: various proteins such as whey protein, casein protein, soybean protein, and pea protein, as well as mixtures and breakdown products thereof; amino acids such as leucine, valine, isoleucine, and glutamine; vitamins such as vitamin B6 and vitamin C; creatine; citric acid; fish oil; etc.

The content of *Bifidobacterium* in the food or beverage composition of the present invention is suitably set according the embodiment of the food or beverage composition, but is usually, in the food or beverage composition, preferably within a range of 1 × 10⁴ to 1 × 10¹³ cfu/g or 1 × 10⁴ to 1 × 10¹³ cfu/ml, and more preferably within a range of 1 × 10⁷ to 1 × 10¹¹ cfu/g or 1 × 10⁷ to 1 × 10¹¹ cfu/ml. "cfu" stands for colony forming unit. In the case where the *Bifidobacterium* are killed bacteria, cfu/g or cfu/ml can be replaced with the number of cells/g or the number of cells/ml.

The food or beverage composition of the present invention may be ingested alone, or may also be ingested together with another food or beverage composition or another food or beverage. For example, in the above embodiment 1, the composition may be ingested together with a food or beverage composition, a food or beverage, or the like for preventing or treating other diseases or symptoms caused by *Fusobacterium.* In addition, in the above embodiment 2, the composition may be ingested together with a food or beverage composition, a food or beverage, or the like for preventing or treating a disease or symptom caused by inflammation of mucosal cells and selected from the group consisting of ulcerative colitis, colon cancer, esophageal cancer, premature delivery, and periodontosis.

The food or beverage composition of the present invention can be sold, in the above embodiment 1, as a food or beverage composition or a food or beverage, which has a label stating the intended use for the prevention or treatment of a disease or symptom caused by *Fusobacterium.* In addition, the composition can be a food or beverage composition or a food or beverage, which has a label stating "for the prevention or treatment of a disease or symptom caused by *Fusobacterium",* for example. In addition, besides these examples, needless to say, any words expressing secondary effects resulting from suppressed adsorption of *Fusobacterium* to mucosal cells can be used.

In the above embodiment 2, the composition can be sold as a food or beverage composition or a food or beverage, which has a label stating the intended use for the prevention or treatment of a disease or symptom caused by inflammation of mucosal cells and selected from the group consisting of ulcerative colitis, colon cancer, esophageal cancer, premature delivery, and periodontosis. In addition, the composition can be a food or beverage composition or a food or beverage, which has a label stating "for the prevention or treatment of a disease or symptom caused by inflammation of mucosal cells and selected from the group consisting of ulcerative colitis, colon cancer, esophageal cancer, premature delivery, and periodontosis", for example. In addition, besides these examples, needless to say, any words can be used as long as they express secondary effects resulting from suppressed involvement of *Fusobacterium* in inflammation of mucosal cells, suppressed adsorption of *Fusobacterium* to mucosal cells, or the suppression of inflammation of mucosal cells by the suppression of adsorption.

In addition, the food or beverage composition of the present invention can be provided/sold as a food or beverage composition or a food or beverage, which has a label stating the intended use such as a probiotic use (including health uses). In addition, the composition can be provided/sold with a label stating, as subjects to ingest the food or beverage composition or the food or beverage, "those who desire a life with bifidobacteria", "those who want to improve the intestinal environment", "those who want to regulate the stomach conditions", "those who want to form an excellent intestinal environment", and the like.

The above "label" means all acts for making consumers aware of the above intended use. As long as the label allows consumers to assume or infer the intended use, all such acts fall within the "label" of the invention regardless of the purpose of the label, the content of the label, the object/medium to be labeled, and the like. However, it is preferable that the label has an expression that allows consumers to directly recognize the intended use.

Specific examples thereof are the following acts: the intended use is described on a product related to the food or beverage composition or the food or beverage of the present invention or on the package thereof; a product stating the intended use thereon or on the package thereof is transferred, delivered, displayed for transfer or delivery, or imported; the intended use is described in an advertisement, price list, or transaction document related to the product and displayed or distributed, or the intended use is described in information having such contents and provided through an electromagnetic means (Internet, etc.); and the like. It is particularly preferable that the label is attached to packages, containers, catalogs, pamphlets, POPs and like advertising materials at the sales location, or other documents.

In addition, as the label, a label that has been permitted by the government or the like (e.g., a label that has been approved based on systems established by the government and attached in an embodiment based on the approval) is preferable. Examples thereof include labels for foods with health claims, more specifically for foods with health claims, health foods, functional foods, enteral foods, special purpose foods, foods with nutrient function claims, quasi drugs, and the like. In addition, labels approved by the Consumer Affairs Agency, such as labels for specified health uses, foods with nutrient function claims, and foods with function claims, as well as labels approved based on a similar system, can be mentioned. Examples of the latter include labels for foods for specified health uses, labels for qualified foods for specified health uses, labels to inform that the body structure or function may be affected, labels to show disease risk reduction, and labels to show evidence-based functionality. More specifically, labels for foods for specified health uses (particularly health use claims) under Cabinet Office Ordinance on Permission for Special Use Claims, Etc., prescribed in Health Promotion Act (August 31, 2009; Cabinet Office Order No. 57), similar labels, and the like can be mentioned.

Further, as an example of the food or beverage composition of the present invention, a baby milk (e.g., an infant formula, etc.) can be mentioned. The "baby milk" refers to a food intended for babies preferably 0 to 36 months old, more preferably 0 to 12 months old, to drink as a substitute for mother's milk, which by itself satisfies the nutritional requirements of babies. The "baby milk" may contain, for example, one or more *Bifidobacterium* as probiotics; prebiotics such as human milk oligosaccharides, fructooligosaccharides, and galactooligosaccharides; proteins derived from casein, soybean, whey, and skimmed milk; carbohydrates such as lactose, saccharose, maltodextrin, starch, and mixtures thereof; lipids (e.g., palm olein, sunflower oil, sunflower oil); and vitamins, minerals, and the like essential for everyday foods. In addition, one or more kinds selected from these groups can be contained.

The pharmaceutical composition of the present invention contains *Bifidobacterium.* As the pharmaceutical composition of the present invention, the *Bifidobacterium* may be directly used, or may also be formulated with a physiologically acceptable liquid or solid pharmaceutical carrier and used.

The dosage form of the pharmaceutical composition of the present invention is not particularly limited, and specific examples thereof include tablets, pills, powders, solutions, suspensions, emulsions, granules, capsules, syrups, suppositories, injections, ointments, patches, eye drops, and nasal drops. In addition, for formulation, additives commonly used as pharmaceutical carriers, such as excipients, binders, disintegrators, lubricants, stabilizers, flavoring agents, diluents, surfactants, and solvents for injection, can be used.

In addition, as the pharmaceutical carriers, any of various organic or inorganic carriers can be used according to the dosage form. Examples of carriers for solid formulations include excipients, binders, disintegrators, lubricants, stabilizers, and flavoring agents.

Examples of excipients include sugar derivatives such as lactose, sucrose, glucose, mannite, and sorbitol; starch derivatives such as corn starch, potato starch, α-starch, dextrin, and carboxymethyl starch; cellulose derivatives such as crystalline cellulose, hydroxypropyl cellulose, hydroxypropylmethyl cellulose, carboxymethyl cellulose, and carboxymethyl cellulose calcium; gum arabic; dextran; pullulan; silicate derivatives such as light anhydrous silicic acid, synthetic aluminum silicate, and magnesium aluminometasilicate; phosphate derivatives such as calcium phosphate; carbonate derivatives such as calcium carbonate; and sulfate derivatives such as calcium sulfate.

Examples of binders include, in addition to the above excipients, gelatin; polyvinylpyrrolidone; and macrogol.

Examples of disintegrators include, in addition to the above excipients, chemically modified starch or cellulose derivatives, such as cross carmellose sodium, sodium carboxy methyl starch, and crosslinked polyvinylpyrrolidone.

Examples of lubricants include talc; stearic acid; metal stearates such as calcium stearate and magnesium stearate; colloidal silica; waxes such as Peegum and spermaceti; boric acid; glycol; carboxylic acids such as fumaric acid and adipic acid; sodium carboxylates such as sodium benzoate; sulfates such as sodium sulfate; leucine; lauryl sulfates such as sodium lauryl sulfate and magnesium lauryl sulfate; silicic acids such as silicic anhydride and silicic acid hydrate; and starch derivatives.

Examples of stabilizers include p-hydroxybenzoate esters such as methylparaben and propylparaben; alcohols such as chlorobutanol, benzyl alcohol, and phenyl ethyl alcohol; benzalkonium chloride; acetic anhydride; and sorbic acid.

Examples of flavoring agents include sweeteners, acidulants, and flavors.

Incidentally, as carriers used for solutions for oral administration, solvents such as water, flavoring agents, and the like can be mentioned.

The content of *Bifidobacterium* in the pharmaceutical composition of the present invention is suitably set according to the dosage form, the usage, the age and sex of the subject, the kind of disease, the degree of disease, other conditions, and the like, but is usually preferably within a range of 1 × 10⁴ to 1 × 10¹³ cfu/g or 1 × 10⁴ to 1 × 10¹³ cfu/ml, and more preferably within a range of 1 × 10⁷ to 1 × 10¹¹ cfu/g or 1 × 10⁷ to 1 × 10¹¹ cfu/ml. In the case where the *Bifidobacterium* are killed bacteria, cfu/g or cfu/ml can be replaced with the number of cells/g or the number of cells/ml.

The timing of administration of the pharmaceutical composition of the present invention is not particularly limited. According to the method for treating the target disease or symptom, the timing of administration can be suitably selected. In addition, the pharmaceutical compositions may be administered prophylactically or may also be used for maintenance therapy. In addition, it is preferable that the dosage form is determined according to the formulation form, the age and sex of the patient, other conditions, the degree of patient's symptoms, and the like. Incidentally, in any case, the pharmaceutical composition of the present invention can be administered once or in several portions a day, and may also be administered once in several days or several weeks. Examples of subjects include humans, cows, sheep, goats, pigs, dogs, cats, and horses.

The pharmaceutical composition according to the above embodiment 1 has a preventing action on the adsorption of *Fusobacterium* to mucosal cells, and thus can be preferably used for preventing or treating diseases or symptoms that can be prevented or treated by preventing the adsorption of *Fusobacterium* to mucosal cells. "Treatment" also includes alleviation and improvement. Specific examples of such applications include the prevention or treatment of diseases or symptoms such as ulcerative colitis, colon cancer, esophageal cancer, premature delivery, and periodontosis. In addition, "premature delivery" herein is defined as a "symptom".

The composition for preventing or treating a disease or symptom caused by inflammation of mucosal cells and selected from the group consisting of ulcerative colitis, colon cancer, esophageal cancer, premature delivery, and periodontosis according to the above embodiment 2 can be utilized as a pharmaceutical composition as described above. The inflammation of mucosal cells is preferably inflammation of mucosal cells involving *Fusobacterium,* and more preferably inflammation of mucosal cells caused by the adsorption of *Fusobacterium* to mucosal cells.

The pharmaceutical composition of the present invention may be administered alone, or may also be used together with another pharmaceutical composition or medicine. For example, in the above embodiment 1, the composition may be used together with a pharmaceutical composition or a medicine for preventing or treating other diseases or symptoms caused by *Fusobacterium,* a pharmaceutical composition or medicine against colon cancer or esophageal cancer (e.g., an anticancer drug, etc.), a digestive medicine, a laxative, a painkiller; a pharmaceutical composition or medicine against ulcerative colitis, a pharmaceutical composition or medicine against periodontosis, or the like. In addition, in the above embodiment 2, the composition may be used together with a pharmaceutical composition, a medicine, or the like for preventing or treating a disease or symptom caused by inflammation of mucosal cells and selected from the group consisting of ulcerative colitis, colon cancer, esophageal cancer, premature delivery, and periodontosis.

Another embodiment of the present invention is use of *Bifidobacterium* in producing a composition for preventing the adsorption of *Fusobacterium* to mucosal cells.

In addition, another embodiment of the present invention is *Bifidobacterium* for use in preventing the adsorption of *Fusobacterium* to mucosal cells.

In addition, another embodiment of the present invention is a method for preventing the adsorption of *Fusobacterium* to mucosal cells, the method including a step of administering *Bifidobacterium* to an application subject or a step of administering the composition for preventing the adsorption of *Fusobacterium* to mucosal cells of the present invention to an application subject.

In addition, another embodiment of the present invention is a method for preventing or treating a disease or symptom caused by *Fusobacterium,* the method including a step of administering *Bifidobacterium* to an application subject or a step of administering the composition for preventing the adsorption of *Fusobacterium* to mucosal cells of the invention to an application subject.

In addition, another embodiment of the present invention is use of *Bifidobacterium* in producing a pharmaceutical composition or a food or beverage composition for preventing or treating a disease or symptom caused by inflammation of mucosal cells and selected from the group consisting of ulcerative colitis, colon cancer, esophageal cancer, premature delivery, and periodontosis.

In addition, another embodiment of the present invention is *Bifidobacterium* for use in preventing or treating a disease or symptom caused by inflammation of mucosal cells and selected from the group consisting of ulcerative colitis, colon cancer, esophageal cancer, premature delivery, and periodontosis.

In addition, another embodiment of the present invention is a method for preventing or treating a disease or symptom caused by inflammation of mucosal cells and selected from the group consisting of ulcerative colitis, colon cancer, esophageal cancer, premature delivery, and periodontosis, the method including a step of administering *Bifidobacterium* to an application subject or a step of administering the pharmaceutical composition or food or beverage composition of the present invention to an application subject.

In addition, another embodiment of the present invention is a pharmaceutical composition or a food or beverage composition containing *Bifidobacterium* as an active ingredient, which has function claims related to the prevention of a disease or symptom caused by inflammation of mucosal cells and selected from the group consisting of ulcerative colitis, colon cancer, esophageal cancer, premature delivery, and periodontosis. The function claims are not particularly limited, and examples thereof include "prevention of ulcerative colitis", "reduction of the risk of development of ulcerative colitis", "reduction of the risk of onset of ulcerative colitis", "prevention of colon cancer", "reduction of the risk of development of colon cancer", "reduction of the risk of onset of colon cancer", "prevention of esophageal cancer", "reduction of the risk of development of esophageal cancer", "reduction of the risk of onset of esophageal cancer", "prevention of premature delivery", "reduction of the risk of premature delivery", "prevention of periodontosis", "reduction of the risk of development of periodontosis", and "reduction of the risk of onset of periodontosis". As used herein, the function claim may be attached to the composition itself or may also be attached to the container or package of the composition.

### EXAMPLES

Hereinafter, the present invention will be described in further detail using examples. However, the present invention is not limited to these examples.

### [Test Example 1]

Preventing Effect of Bacteria Belonging to *Bifidobacterium longum subspecies longum* on Adsorption of *Fusobacterium* to Mucosal Cells

Colon cancer cell strain HCT116 cells (5 × 10⁵ cells) were suspended in 0.1 ml of PBS. From the absorbance at 660 nm of each of bacterial culture solutions of *Bifidobacterium breve* ATCC 15700 and *Bifidobacterium longum subsp. longum* NITE BP-02572, NITE BP-02573, and NITE BP-02574 separately cultured in a GAM medium (GAM Broth "Nissui", manufactured by Nissui Pharmaceutical Co., Ltd.) at 37°C for 16 hours, the number of bacteria was calculated, and, per 5 × 10⁵ HCT116 cells, 2.5 × 10⁸ CFU each bacterial cells corresponding to MOI 500 were dissolved in 0.1 ml of PBS. Subsequently, 5 × 10⁵ HCT116 cells were allowed to react (coexist) with 2.5 × 10⁸ CFU each bacterial cells in a 50 mM phosphate buffer (pH 5.0) at 37°C for 3 hours, followed by washing with 1 ml of PBS containing 1% BSA three times. Further, 1 × 10¹⁰ CFU *Fusobacterium nucleatum* (ATCC 23726) separately cultured in a GAM medium (GAM Broth "Nissui", manufactured by Nissui Pharmaceutical Co., Ltd.) at 37°C for 16 hours was FITC-labeled using a FITC isomer (sigma F7250), followed by washing with PBS repeatedly four times, and then 2.5 × 10⁷ CFU bacterial cells corresponding to MOI 50 were dissolved in 0.02 ml of PBS. The above HCT116 cells allowed to react (coexist) with each bacterial cells were allowed to react (coexist) with 2.5 × 10⁷ CFU FITC-labeled *Fusobacterium nucleatum* (ATCC 23726) at room temperature for 1 hour, followed by washing with PBS containing 1% BSA repeatedly three times. Subsequently, regarding FITC-positive cells as cells having adsorbed thereto FITC-labeled *Fusobacterium nucleatum* (ATCC 23726), the proportion of FITC-positive cells in the HCT116 cells treated with each bacterial cells was calculated using Flowcytometer (BD FACSCanto, manufactured by Becton, Dickinson and Company). The results are shown in Table 1.

As shown by the following results, the FITC-positive cell percentage in the HCT116 cells untreated with bacterial cells was 9.89%. Meanwhile, in the cells treated with *Bifidobacterium breve* ATCC 15700, the FITC-positive cell percentage was 8.36%, that is, not much different, but in the cells treated with *Bifidobacterium longum subsp. longum* NITE BP-02572, NITE BP-02573, and NITE BP-02574, the FITC-positive cell percentages decreased to 1.19%, 2.04%, and 0.60%, respectively.

### [Table 1]

**Table 1**

| Strain used to treat HCT116 cells | Proportion of FITC-positive cells |
|---|---|
| Untreated | 9.89 % |
| *Bifidobacterium breve* ATCC 15700 | 8.36 % |
| *Bifidobacterium longum subsp. longum* NITE BP-02572 | 1.19 % |
| *Bifidobacterium longum subsp. longum* NITE BP-02573 | 2.04 % |
| *Bifidobacterium longum subsp. longum* NITE BP-02574 | 0.60 % |

### [Test Example 2]

Preventing Effect of Bacteria Belonging to *Bifidobacterium bifidum* on Adsorption of *Fusobacterium* to Mucosal Cells

Colon cancer cell strain HCT116 cells (5 × 10⁵ cells) were suspended in 0.1 ml of PBS. From the absorbance at 660 nm of each of bacterial culture solutions of *Bifidobacterium breve* ATCC 15700 and *Bifidobacterium bifidum* NITE BP-1252, NITE BP-02570, and NITE BP-02571 separately cultured in a GAM medium (GAM Broth "Nissui", manufactured by Nissui Pharmaceutical Co., Ltd.) at 37°C for 16 hours, the number of bacteria was calculated, and, per 5 × 10⁵ HCT116 cells, 1.5 × 10⁸ CFU each bacterial cells corresponding to MOI 300 were dissolved in 0.1 ml of PBS. Subsequently, 5 × 10⁵ HCT116 cells were allowed to react (coexist) with 2.5 × 10⁸ CFU each bacterial cells in a 50 mM phosphate buffer (pH 5.0) at 37°C for 3 hours, followed by washing with 1 ml of PBS containing 1% BSA three times. Further, 1 × 10¹⁰ CFU *Fusobacterium nucleatum* (ATCC 23726) separately cultured in a GAM medium (GAM Broth "Nissui", manufactured by Nissui Pharmaceutical Co., Ltd.) at 37°C for 16 hours was FITC-labeled using a FITC isomer (sigma F7250), followed by washing with PBS repeatedly four times, and then 2.5 × 10⁷ CFU bacterial cells corresponding to MOI 50 were dissolved in 0.02 ml of PBS. The above HCT116 cells allowed to react (coexist) with each bacterial cells were allowed to react (coexist) with 2.5 × 10⁷ CFU FITC-labeled *Fusobacterium nucleatum* (ATCC 23726) at room temperature for 1 hour, followed by washing with PBS containing 1% BSA repeatedly three times. Subsequently, regarding FITC-positive cells as cells having adsorbed thereto FITC-labeled *Fusobacterium nucleatum* (ATCC 23726), the proportion of FITC-positive cells in the HCT116 cells treated with each bacterial cells was calculated using Flowcytometer (BD FACSCanto, manufactured by Becton, Dickinson and Company). The results are shown in Table 2.

As shown by the following results, the FITC-positive cell percentage in the HCT116 cells untreated with bacterial cells was 7.09%. Meanwhile, in the cells treated with *Bifidobacterium breve* ATCC 15700, the FITC-positive cell percentage was 7.67%, that is, not much different, but in the cells treated with *Bifidobacterium bifidum* NITE BP-1252, NITE BP-02570, and NITE BP-02571, the FITC-positive cell percentages decreased to 3.03%, 2.17%, and 2.85%, respectively.

### [Table 2]

**Table 2**

| Strain used to treat HCT116 cells | Proportion of FITC-positive cells |
|---|---|
| Untreated | 7.09 % |
| *Bifidobacterium breve* ATCC 15700 | 7.67 % |
| *Bifidobacterium bifidum* NITE BP-1252 | 3.03 % |
| *Bifidobacterium bifidum* NITE BP-02570 | 2.17 % |
| *Bifidobacterium bifidum* NITE BP-02571 | 2.85 % |

### [Test Example 3]

Preventing Effect of Bacteria Belonging to *Bifidobacterium longum subspecies infantis* on Adsorption of *Fusobacterium* to Mucosal Cells

Colon cancer cell strain HCT116 cells (5 × 10⁵ cells) were suspended in 0.1 ml of PBS. From the absorbance at 660 nm of each of bacterial culture solutions of *Bifidobacterium breve* ATCC 15700 and *Bifidobacterium longum subsp. infantis* ATCC 15697 separately cultured in a GAM medium (GAM Broth "Nissui", manufactured by Nissui Pharmaceutical Co., Ltd.) at 37°C for 16 hours, the number of bacteria was calculated, and, per 5 × 10⁵ HCT116 cells, 2.5 × 10⁷ CFU each bacterial cells corresponding to MOI 50 were dissolved in 0.1 ml of PBS. Subsequently, 5 × 10⁵ HCT116 cells were allowed to react (coexist) with 2.5 × 10⁷ CFU each bacterial cells in a 50 mM phosphate buffer (pH 5.0) at 37°C for 2 hours, followed by washing with 1 ml of PBS containing 1% BSA three times. Further, 1 × 10¹⁰ CFU *Fusobacterium nucleatum* (ATCC 23726) separately cultured in a GAM medium (GAM Broth "Nissui", manufactured by Nissui Pharmaceutical Co., Ltd.) at 37°C for 16 hours was FITC-labeled using a FITC isomer (sigma F7250), followed by washing with PBS repeatedly four times, and then 5 × 10⁶ CFU bacterial cells corresponding to MOI 10 were dissolved in 0.02 ml of PBS. The above HCT116 cells allowed to react (coexist) with each bacterial cells were allowed to react (coexist) with 5 × 10⁶ CFU FITC-labeled *Fusobacterium nucleatum* (ATCC 23726) at room temperature for 1 hour, followed by washing with PBS containing 1% BSA repeatedly three times. Subsequently, regarding FITC-positive cells as cells having adsorbed thereto FITC-labeled *Fusobacterium nucleatum* (ATCC 23726), the proportion of FITC-positive cells in the HCT116 cells treated with each bacterial cells was calculated using Flowcytometer (BD FACSCanto, manufactured by Becton, Dickinson and Company). The results are shown in Table 3.

As shown by the following results, the FITC-positive cell percentage in the HCT116 cells untreated with bacterial cells was 7.93%. Meanwhile, in the cells treated with *Bifidobacterium breve* ATCC 15700, the FITC-positive cell percentage was 8.61%, that is, not much different, but in the cells treated with *Bifidobacterium longum subsp. infantis* ATCC 15697, the FITC-positive cell percentage decreased to 3.52%.

### [Table 3]

**Table 3**

| Strain used to treat HCT116 cells | Proportion of FITC-positive cells |
|---|---|
| Untreated | 7.93 % |
| *Bifidobacterium breve* ATCC 15700 | 8.61 % |
| *Bifidobacterium longum subsp. infantis* ATCC 15697 | 3.52 % |

### [Production Example 1]

*Bifidobacterium longum subsp. longum* NITE BP-02572, NITE BP-02573, NITE BP-02574, *Bifidobacterium bifidum* NITE BP-1252, NITE BP-02570, NITE BP-02571, and *Bifidobacterium longum subsp. infantis* ATCC 15697 are each added to 3 mL of MRS liquid media and anaerobically cultured at 37°C for 16 hours, and the culture solution is concentrated and lyophilized into a powder of each kind of bacteria (bacterial powder). Each bacterial powder is uniformly mixed with a whey protein concentrate (WPC) into a composition. 20 g of the composition is dissolved in 200 g of water, thereby giving a composition for preventing adsorption. When the composition is orally administered, a preventing effect on the adsorption of *Fusobacterium* to colon epithelial cells, vascular endothelial cells of the uterus, mucosal epithelial cells of the esophagus, and mucosal cells of the gingiva can be expected.

### [Production Example 2]

*Bifidobacterium longum subsp. longum* NITE BP-02572, NITE BP-02573, NITE BP-02574, *Bifidobacterium bifidum* NITE BP-1252, NITE BP-02570, NITE BP-02571, and *Bifidobacterium longum subsp. infantis* ATCC 15697 are each added to 3 mL of MRS liquid media and anaerobically cultured at 37°C for 16 hours, and the culture solution is concentrated and lyophilized into a powder of each kind of bacteria (bacterial powder). Each bacterial powder is uniformly mixed with a dry powder of a milk protein concentrate (MPC 480, manufactured by Fonterra, protein content: 80 mass%, casein protein:whey protein = about 8:2) into a composition. 20 g of the composition is dissolved in 200 g of water, thereby giving a composition for preventing adsorption. When the composition is orally administered, a preventing effect on the adsorption of *Fusobacterium* to colon epithelial cells, vascular endothelial cells of the uterus, mucosal epithelial cells of the esophagus, and mucosal cells of the gingiva can be expected.

### [Production Example 3]

*Bifidobacterium longum subsp. longum* NITE BP-02572, NITE BP-02573, NITE BP-02574, *Bifidobacterium bifidum* NITE BP-1252, NITE BP-02570, NITE BP-02571, and *Bifidobacterium longum subsp. infantis* ATCC 15697 are each added to 3 mL of MRS liquid media and anaerobically cultured at 37°C for 16 hours, and the culture solution is concentrated and lyophilized into a powder of each kind of bacteria (bacterial powder). Next, crystalline cellulose is fed to a stirring granulator and mixed. Subsequently, purified water is added to perform granulation, and the resulting granules are dried, thereby giving granules containing each bacterial extract and containing an excipient. When such a composition is orally administered, a preventing effect on the adsorption of *Fusobacterium* to colon epithelial cells, vascular endothelial cells of the uterus, mucosal epithelial cells of the esophagus, and mucosal cells of the gingiva can be expected.

### [Production Example 4]

At least one or two kinds of *Bifidobacterium longum subsp. longum* NITE BP-02572, NITE BP-02573, and NITE BP-02574 is added to 3 mL of MRS liquid media and anaerobically cultured at 37°C for 16 hours, and the culture solution is concentrated and lyophilized into a powder of the bacteria (bacterial powder). The bacterial powder is uniformly mixed with an oligosaccharide into a composition. The composition is served to elderly people as a liquid food for elderly people. The composition is served every day at breakfast for 1 week such that the intake of *Bifidobacterium* is 1 × 10⁸ to 1 × 10¹⁰ CFU/kg body weight/day. In the case where the *Bifidobacterium* are killed bacteria, CFU/kg body weight/day can be replaced with the number of cells/kg body weight/day. In addition, the bacterial powder may also be mixed with a food or drink, such as a fermented milk. Examples of usable oligosaccharides are isomaltooligosaccharides, lactulose, raffinose, fructooligosaccharides, galactooligosaccharides, soybean oligosaccharides, and human milk oligosaccharides (HMOs).

Examples of human milk oligosaccharides include sialic acid, 2'-fucosyllactose, 3-fucosyllactose, 2',3-difucosyllactose, 3'-sialyllactose, 6'-sialyllactose, 3-fucosyl-3'-sialyllactose, lacto-N-tetraose, lacto-N-neotetraose, lacto-N-fucopentaose I, lacto-N-fucopentaose II, lacto-N-fucopentaose III, lacto-N-fucopentaose V, lacto-N-difucosylhexaose I, lacto-N-difucosylhexaose II, lacto-N-sialylpentaose, LSTa, LSTb, and LSTc.

When such a composition is orally administered, a preventing effect on the adsorption of *Fusobacterium* to colon epithelial cells, vascular endothelial cells of the uterus, mucosal epithelial cells of the esophagus, and mucosal cells of the gingiva can be expected.

### [Production Example 5]

A composition can be obtained in the same manner as in Production Example 4, except for using at least one or two kinds of *Bifidobacterium bifidum* NITE BP-1252, NITE BP-02570, and NITE BP-02571.

### [Production Example 6]

A composition can be obtained in the same manner as in Production Example 4, except for using *Bifidobacterium longum subsp. infantis* ATCC 15697.

### [Production Example 7]

A method for producing fermented milk having added thereto *Bifidobacterium longum subsp. longum* NITE BP-02572, NITE BP-02573, NITE BP-02574, *Bifidobacterium bifidum* NITE BP-1252, NITE BP-02570, NITE BP-02571, and *Bifidobacterium longum subsp. infantis* ATCC 15697, respectively, will be shown below.

First, a milk raw material is mixed with water, other components, and the like as necessary, preferably followed by a homogenization treatment, and the mixture is subjected to a heat sterilization treatment. The homogenization treatment and the heat sterilization treatment can be performed in the usual manner. A lactic acid bacteria starter is added (inoculated) to the sterilized liquid milk preparation that has been heat-sterilized, maintained at a predetermined fermentation temperature, and fermented to give a fermentation product. As a result of fermentation, a curd is formed.

As lactic acid bacteria starters, for example, lactic acid bacteria commonly used for yogurt manufacture, such as Lactobacillus bulgaricus, Lactococcus lactis, and Streptococcus thermophilus, can be used. When the pH reaches the desired value, the formed curd is crushed by stirring and cooled to 10°C or less to give a fermentation product. As a result of cooling to 10°C or less, the activity of lactic acid bacteria can be reduced to suppress the generation of acids.

Next, the fermentation product obtained in the fermentation step is subjected to a heating treatment to give a heated fermentation product (a fermentation product after a heating treatment). As a result of moderately heating the fermentation product, the generation of acids by lactic acid bacteria in the heated fermentation product can be suppressed. Accordingly, a decrease in pH during the subsequent production steps and/or during the storage of the bifidobacteria-containing concentrated fermented milk can be suppressed, and, as a result, the survivability of bifidobacteria can be improved.

Next, to the heated fermentation product obtained in the heating treatment step, the *Bifidobacterium* of the present invention are added. The added amount of the *Bifidobacterium* is preferably 1 × 10⁷ to 1 × 10¹¹ CFU/ml, more preferably 1 × 10⁸ to 1 × 10¹⁰ CFU/ml, relative to the heated fermentation product. In the case where the *Bifidobacterium* are killed bacteria, CFU/ml can be replaced with the number of cells/ml.

The addition of the *Bifidobacterium* to the heated fermentation product is followed by concentration. The concentration step can be performed suitably using a known condensation method. For example, a centrifugal separation method or a membrane separation method can be used.

In the centrifugal separation method, whey in the material to be concentrated (the heated fermentation product having added thereto bifidobacteria) is removed, whereby a bifidobacteria-containing concentrated fermented milk having an increased solids content can be obtained.

When the fermented milk obtained as above is ingested, a preventing effect on the adsorption of *Fusobacterium* to colon epithelial cells, vascular endothelial cells of the uterus, mucosal epithelial cells of the esophagus, and mucosal cells of the gingiva can be expected.

## Claims

1. A composition for preventing the adsorption of *Fusobacterium* to mucosal cells, comprising *Bifidobacterium* as an active ingredient.

2. The composition for preventing adsorption according to claim 1, wherein the mucosal cells are colon epithelial cells, mucosal epithelial cells of the esophagus, vascular endothelial cells of the uterus, or mucosal cells of the gingiva.

3. The composition for adsorption suppression according to claim 1 or 2, wherein the *Bifidobacterium* are at least one kind of bacteria selected from the group consisting of bacteria belonging to *Bifidobacterium longum subspecies longum,* bacteria belonging to *Bifidobacterium bifidum,* and bacteria belonging to *Bifidobacterium longum subspecies infantis.*

4. The composition for preventing adsorption according to claim 3, wherein the bacteria belonging to *Bifidobacterium longum subspecies longum* are at least one kind of bacteria selected from the group consisting of *Bifidobacterium longum subspecies longum* NITE BP-02572, NITE BP-02573, and NITE BP-02574.

5. The composition for adsorption suppression according to claim 3 or 4, wherein the bacteria belonging to *Bifidobacterium bifidum* are at least one kind of bacteria selected from the group consisting of *Bifidobacterium bifidum* NITE BP-1252, NITE BP-02570, and NITE BP-02571.

6. The composition for preventing adsorption according to any one of claims 3 to 5, wherein the bacteria belonging to *Bifidobacterium longum subspecies infantis* are *Bifidobacterium longum subspecies infantis* ATCC 15697.

7. A pharmaceutical composition comprising *Bifidobacterium* as an active ingredient, for preventing or treating a disease or symptom caused by inflammation of mucosal cells and selected from the group consisting of ulcerative colitis, colon cancer, esophageal cancer, premature delivery, and periodontosis.

8. The pharmaceutical composition according to claim 7, wherein the inflammation of mucosal cells is inflammation of mucosal cells involving *Fusobacterium.*

9. The pharmaceutical composition according to claim 7 or 8, wherein the *Bifidobacterium* are at least one kind of bacteria selected from the group consisting of bacteria belonging to *Bifidobacterium longum subspecies longum,* bacteria belonging to *Bifidobacterium bifidum,* and bacteria belonging to *Bifidobacterium longum subspecies infantis.*

10. The pharmaceutical composition according to claim 9, wherein the bacteria belonging to *Bifidobacterium longum subspecies longum* are at least one kind of bacteria selected from the group consisting of *Bifidobacterium longum subspecies longum* NITE BP-02572, NITE BP-02573, and NITE BP-02574.

11. The pharmaceutical composition according to claim 9 or 10, wherein the bacteria belonging to *Bifidobacterium bifidum* are at least one kind of bacteria selected from the group consisting of *Bifidobacterium bifidum* NITE BP-1252, NITE BP-02570, and NITE BP-02571.

12. The pharmaceutical composition according to any one of claims 9 to 11, wherein the bacteria belonging to *Bifidobacterium longum subspecies infantis* are *Bifidobacterium longum subspecies infantis* ATCC 15697.

13. A food or beverage composition comprising *Bifidobacterium* as an active ingredient, for preventing or treating a disease or symptom caused by inflammation of mucosal cells and selected from the group consisting of ulcerative colitis, colon cancer, esophageal cancer, premature delivery, and periodontosis.

14. The food or beverage composition according to claim 13, wherein the inflammation of mucosal cells is inflammation of mucosal cells involving *Fusobacterium.*

15. The food or beverage composition according to claim 13 or 14, wherein the *Bifidobacterium* are at least one kind of bacteria selected from the group consisting of bacteria belonging to *Bifidobacterium longum subspecies longum,* bacteria belonging to *Bifidobacterium bifidum,* and bacteria belonging to *Bifidobacterium longum subspecies infantis.*

16. The food or beverage composition according to claim 15, wherein the bacteria belonging to *Bifidobacterium longum subspecies longum* are at least one kind of bacteria selected from the group consisting of *Bifidobacterium longum subspecies longum* NITE BP-02572, NITE BP-02573, and NITE BP-02574.

17. The food or beverage composition according to claim 15 or 16, wherein the bacteria belonging to *Bifidobacterium bifidum* are at least one kind of bacteria selected from the group consisting of *Bifidobacterium bifidum* NITE BP-1252, NITE BP-02570, and NITE BP-02571.

18. The food or beverage composition according to any one of claims 15 to 17, wherein the bacteria belonging to *Bifidobacterium longum subspecies infantis* are *Bifidobacterium longum subspecies infantis* ATCC 15697.

19. *Bifidobacterium longum subspecies longum* NITE BP-02572.

20. *Bifidobacterium longum subspecies longum* NITE BP-02573.

21. *Bifidobacterium longum subspecies longum* NITE BP-02574.

22. *Bifidobacterium bifidum* NITE BP-02570.

23. *Bifidobacterium bifidum* NITE BP-02571.


SEQUENCE LISTING

<110>  Morinaga Milk Industry Co., Ltd.

<120>  A composition comprising Bifidobacterium bacteria as an active
       ingredient

<130>  FP290-18511

<150>  JP 2017-237465
<151>  2017-12-12

<160>  5

<170>  PatentIn version 3.5

<210>  1
<211>  1536
<212>  DNA
<213>  Bifidobacterium longum

<400>  1
tttttgtgga gggttcgatt ctggctcagg atgaacgctg gcggcgtgct taacacatgc      60

aagtcgaacg ggatccatca ggctttgctt ggtggtgaga gtggcgaacg ggtgagtaat     120

gcgtgaccga cctgccccat acaccggaat agctcctgga aacgggtggt aatgccggat     180

gctccagttg atcgcatggt cttctgggaa agctttcgcg gtatgggatg gggtcgcgtc     240

ctatcagctt gacggcgggg taacggccca ccgtggcttc gacgggtagc cggcctgaga     300

gggcgaccgg ccacattggg actgagatac ggcccagact cctacgggag gcagcagtgg     360

ggaatattgc acaatgggcg caagcctgat gcagcgacgc cgcgtgaggg atggaggcct     420

tcgggttgta aacctctttt atcggggagc aagcgagagt gagtttaccc gttgaataag     480

caccggctaa ctacgtgcca gcagccgcgg taatacgtag ggtgcaagcg ttatccggaa     540

ttattgggcg taaagggctc gtaggcggtt cgtcgcgtcc ggtgtgaaag tccatcgctt     600

aacggtggat ccgcgccggg tacgggcggg cttgagtgcg gtaggggaga ctggaattcc     660

cggtgtaacg gtggaatgtg tagatatcgg gaagaacacc aatggcgaag gcaggtctct     720

gggccgttac tgacgctgag gagcgaaagc gtggggagcg aacaggatta gataccctgg     780

tagtccacgc cgtaaacggt ggatgctgga tgtggggccc gttccacggg ttccgtgtcg     840

gagctaacgc gttaagcatc ccgcctgggg agtacggccg caaggctaaa actcaaagaa     900

attgacgggg gcccgcacaa gcggcggagc atgcggatta attcgatgca acgcgaagaa     960

ccttacctgg gcttgacatg ttcccgacgg tcgtagagat acggcttccc ttcggggcgg    1020

gttcacaggt ggtgcatggt cgtcgtcagc tcgtgtcgtg agatgttggg ttaagtcccg    1080

caacgagcgc aaccctcgcc ccgtgttgcc agcggattat gccgggaact cacgggggac    1140

cgccggggtt aactcggagg aaggtgggga tgacgtcaga tcatcatgcc ccttacgtcc    1200

agggcttcac gcatgctaca atggccggta caacgggatg cgacgcggcg acgcggagcg    1260

gatccctgaa aaccggtctc agttcggatc gcagtctgca actcgactgc gtgaaggcgg 1320

agtcgctagt aatcgcgaat cagcaacgtc gcggtgaatg cgttcccggg ccttgtacac 1380

accgcccgtc aagtcatgaa agtgggcagc acccgaagcc ggtggcctaa ccccttgtgg 1440

gatggagccg tctaaggtga ggctcgtgat tgggactaag tcgtaacaag gtagccgtac 1500

cggaaggtgc ggctggatca cctcctttct acggag 1536

<210> 2
<211> 1536
<212> DNA
<213> Bifidobacterium longum

<400> 2
tttttgtgga gggttcgatt ctggctcagg atgaacgctg gcggcgtgct taacacatgc 60

aagtcgaacg ggatccaccg ggctttgctt ggtggtgaga gtggcgaacg ggtgagtaat 120

gcgtgaccga cctgccccat acaccggaat agctcctgga aacgggtggt aatgccggat 180

gctccagttg atcgcatggt cttctgggaa agctttcgcg gtatgggatg gggtcgcgtc 240

ctatcagctt gacggcgggg taacggccca ccgtggcttc gacgggtagc cggcctgaga 300

gggcgaccgg ccacattggg actgagatac ggcccagact cctacgggag gcagcagtgg 360

ggaatattgc acaatgggcg caagcctgat gcagcgacgc cgcgtgaggg atggaggcct 420

tcgggttgta aacctctttt atcggggagc aagcgagagt gagtttaccc gttgaataag 480

caccggctaa ctacgtgcca gcagccgcgg taatacgtag ggtgcaagcg ttatccggaa 540

ttattgggcg taaagggctc gtaggcggtt cgtcgcgtcc ggtgtgaaag tccatcgctt 600

aacggtggat ccgcgccggg tacgggcggg cttgagtgcg gtaggggaga ctggaattcc 660

cggtgtaacg gtggaatgtg tagatatcgg gaagaacacc aatggcgaag gcaggtctct 720

gggccgttac tgacgctgag gagcgaaagc gtggggagcg aacaggatta gataccctgg 780

tagtccacgc cgtaaacggt ggatgctgga tgtggggccc gttccacggg ttccgtgtcg 840

gagctaacgc gttaagcatc ccgcctgggg agtacggccg caaggctaaa actcaaagaa 900

attgacgggg gcccgcacaa gcggcggagc atgcggatta attcgatgca acgcgaagaa 960

ccttacctgg gcttgacatg ttcccgacgg tcgtagagat acggcttccc ttcggggcgg 1020

gttcacaggt ggtgcatggt cgtcgtcagc tcgtgtcgtg agatgttggg ttaagtcccg 1080

caacgagcgc aaccctcgcc ccgtgttgcc agcggattat gccgggaact cacgggggac 1140

cgccggggtt aactcggagg aaggtgggga tgacgtcaga tcatcatgcc ccttacgtcc 1200

agggcttcac gcatgctaca atggccggta caacgggatg cgacgcggcg acgcggagcg 1260

gatccctgaa aaccggtctc agttcggatc gcagtctgca actcgactgc gtgaaggcgg 1320

agtcgctagt aatcgcgaat cagcaacgtc gcggtgaatg cgttcccggg ccttgtacac 1380

accgcccgtc aagtcatgaa agtgggcagc acccgaagcc ggtggcctaa ccccttgtgg 1440

gatggagccg tctaaggtga ggctcgtgat tgggactaag tcgtaacaag gtagccgtac 1500

cggaaggtgc ggctggatca cctcctttct acggag 1536

<210> 3
<211> 1536
<212> DNA
<213> Bifidobacterium longum

<400> 3
tttttgtgga gggttcgatt ctggctcagg atgaacgctg gcggcgtgct taacacatgc 60

aagtcgaacg ggatccatca ggctttgctt ggtggtgaga gtggcgaacg ggtgagtaat 120

gcgtgaccga cctgccccat acaccggaat agctcctgga aacgggtggt aatgccggat 180

gctccagttg atcgcatggt cttctgggaa agctttcgcg gtatgggatg gggtcgcgtc 240

ctatcagctt gacggcgggg taacggccca ccgtggcttc gacgggtagc cggcctgaga 300

gggcgaccgg ccacattggg actgagatac ggcccagact cctacgggag gcagcagtgg 360

ggaatattgc acaatgggcg caagcctgat gcagcgacgc cgcgtgaggg atggaggcct 420

tcgggttgta aacctctttt atcggggagc aagcgagagt gagtttaccc gttgaataag 480

caccggctaa ctacgtgcca gcagccgcgg taatacgtag ggtgcaagcg ttatccggaa 540

ttattgggcg taaagggctc gtaggcggtt cgtcgcgtcc ggtgtgaaag tccatcgctt 600

aacggtggat ccgcgccggg tacgggcggg cttgagtgcg gtaggggaga ctggaattcc 660

cggtgtaacg gtggaatgtg tagatatcgg gaagaacacc aatggcgaag gcaggtctct 720

gggccgttac tgacgctgag gagcgaaagc gtggggagcg aacaggatta gataccctgg 780

tagtccacgc cgtaaacggt ggatgctgga tgtggggccc gttccacggg ttccgtgtcg 840

gagctaacgc gttaagcatc ccgcctgggg agtacggccg caaggctaaa actcaaagaa 900

attgacgggg gcccgcacaa gcggcggagc atgcggatta attcgatgca acgcgaagaa 960

ccttacctgg gcttgacatg ttcccgacgg tcgtagagat acggcttccc ttcggggcgg 1020

gttcacaggt ggtgcatggt cgtcgtcagc tcgtgtcgtg agatgttggg ttaagtcccg 1080

caacgagcgc aaccctcgcc ccgtgttgcc agcggattat gccgggaact cacgggggac 1140

cgccggggtt aactcggagg aaggtgggga tgacgtcaga tcatcatgcc ccttacgtcc 1200

agggcttcac gcatgctaca atggccggta caacgggatg cgacgcggcg acgcggagcg 1260

gatccctgaa aaccggtctc agttcggatc gcagtctgca actcgactgc gtgaaggcgg 1320

agtcgctagt aatcgcgaat cagcaacgtc gcggtgaatg cgttcccggg ccttgtacac 1380

accgcccgtc aagtcatgaa agtgggcagc acccgaagcc ggtggcctaa ccccttgtgg 1440

gatggagccg tctaaggtga ggctcgtgat tgggactaag tcgtaacaag gtagccgtac 1500

cggaaggtgc ggctggatca cctcctttct acggag 1536

<210> 4
<211> 1434
<212> DNA
<213> Bifidobacterium bifidum

<220>
<221> misc_feature
<222> (504)..(504)
<223> n is a, c, g, or t

<400> 4
gtcgaacggg atccatcaag cttgcttggt ggtgagagtg gcgaacgggt gagtaatgcg 60
tgaccgacct gccccatgct ccggaatagc tcctggaaac gggtggtaat gccggatgtt 120
ccacatgatc gcatgtgatt gtgggaaaga ttctatcggc gtgggatggg gtcgcgtcct 180
atcagcttgt tggtgaggta acggctcacc aaggcttcga cgggtagccg gcctgagagg 240
gcgaccggcc acattgggac tgagatacgg cccagactcc tacgggaggc agcagtgggg 300
aatattgcac aatgggcgca agcctgatgc agcgacgccg cgtgagggat ggaggccttc 360
gggttgtaaa cctcttttgt ttgggagcaa gccttcgggt gagtgtacct ttcgaataag 420
cgccggctaa ctacgtgcca gcagccgcgg taatacgtag ggcgcaagcg ttatccggat 480
ttattgggcg taaagggctc gtangcggct cgtcgcgtcc ggtgtgaaag tccatcgctt 540
aacggtggat ctgcgccggg tacgggcggg ctggagtgcg gtaggggaga ctggaattcc 600
cggtgtaacg gtggaatgtg tagatatcgg gaagaacacc gatggcgaag gcaggtctct 660
gggccgtcac tgacgctgag gagcgaaagc gtggggagcg aacaggatta gataccctgg 720
tagtccacgc cgtaaacggt ggacgctgga tgtggggcac gttccacgtg ttccgtgtcg 780
gagctaacgc gttaagcgtc ccgcctgggg agtacggccg caaggctaaa actcaaagaa 840
attgacgggg gcccgcacaa gcggcggagc atgcggatta attcgatgca acgcgaagaa 900
ccttacctgg gcttgacatg ttcccgacga cgccagagat ggcgtttccc ttcggggcgg 960
gttcacaggt ggtgcatggt cgtcgtcagc tcgtgtcgtg agatgttggg ttaagtcccg 1020
caacgagcgc aaccctcgcc ccgtgttgcc agcacgttat ggtgggaact cacgggggac 1080
cgccggggtt aactcggagg aaggtgggga tgacgtcaga tcatcatgcc ccttacgtcc 1140
agggcttcac gcatgctaca atggccggta cagcgggatg cgacatggcg acatggagcg 1200
gatccctgaa aaccggtctc agttcggatc ggagcctgca acccggctcc gtgaaggcgg 1260
agtcgctagt aatcgcggat cagcaacgcc gcggtgaatg cgttcccggg ccttgtacac 1320
accgcccgtc aagtcatgaa agtgggcagc acccgaagcc ggtggcctaa ccccttgtgg 1380
gatggagccg tctaaggtga ggctcgtgat tgggactaag tcgtaacaag gtaa 1434

<210>  5
<211>  1416
<212>  DNA
<213>  Bifidobacterium bifidum

<220>
<221>  misc_feature
<222>  (1407)..(1407)
<223>  n is a, c, g, or t

<400>  5
ttgcttggtg gtgagagtgg cgaacgggtg agtaatgcgt gaccgacctg ccccatgctc      60

cggaatagct cctggaaacg ggtggtaatg ccggatgttc cacatgatcg catgtgattg     120

tgggaaagat tctatcggcg tgggatgggg tcgcgtccta tcagcttgtt ggtgaggtaa     180

cggctcacca aggcttcgac gggtagccgg cctgagaggg cgaccggcca cattgggact     240

gagatacggc ccagactcct acgggaggca gcagtgggga atattgcaca atgggcgcaa     300

gcctgatgca gcgacgccgc gtgagggatg gaggccttcg ggttgtaaac ctcttttgtt     360

tgggagcaag ccttcgggtg agtgtacctt tcgaataagc gccggctaac tacgtgccag     420

cagccgcggt aatacgtagg gcgcaagcgt tatccggatt tattgggcgt aaagggctcg     480

taggcggctc gtcgcgtccg gtgtgaaagt ccatcgctta acggtggatc tgcgccgggt     540

acgggcgggc tggagtgcgg taggggagac tggaattccc ggtgtaacgg tggaatgtgt     600

agatatcggg aagaacaccg atggcgaagg caggtctctg ggccgtcact gacgctgagg     660

agcgaaagcg tggggagcga acaggattag ataccctggt agtccacgcc gtaaacggtg     720

gacgctggat gtggggcacg ttccacgtgt tccgtgtcgg agctaacgcg ttaagcgtcc     780

cgcctgggga gtacggccgc aaggctaaaa ctcaaagaaa ttgacggggg cccgcacaag     840

cggcggagca tgcggattaa ttcgatgcaa cgcgaagaac cttacctggg cttgacatgt     900

tcccgaggac gccagagatg gcgtttccct tcggggcggg ttcacaggtg gtgcatggtc     960

gtcgtcagct cgtgtcgtga gatgttgggt taagtcccgc aacgagcgca accctcgccc    1020

cgtgttgcca gcacgttatg gtgggaactc acgggggacc gccggggtta actcggagga    1080

aggtggggat gacgtcagat catcatgccc cttacgtcca gggcttcacg catgctacaa    1140

tggccggtac agcgggatgc gacatggcga catggagcgg atccctgaaa accggtctca    1200

gttcggatcg gagcctgcaa cccggctccg tgaaggcgga gtcgctagta atcgcggatc    1260

agcaacgccg cggtgaatgc gttcccgggc cttgtacaca ccgcccgtca agtcatgaaa    1320

gtgggcagca cccgaagccg gtggcctaac cccttgtggg atggagccgt ctaaggtgag    1380

gctcgtgatt gggactaagt cgtaacnagg taacca                              1416